## Europäisches Patentamt
## European Patent Office
## Office européen des brevets

(19)

(11) Veröffentlichungsnummer: **0 483 512 B1**

(12) **EUROPÄISCHE PATENTSCHRIFT**

(45) Veröffentlichungstag der Patentschrift: **22.11.95**

(51) Int. Cl.⁶: **G01N 33/53**, G01N 33/543, G01N 33/577

(21) Anmeldenummer: **91116392.1**

(22) Anmeldetag: **26.09.91**

(54) **Immunologisches Präzipitationsverfahren zur Bestimmung eines bindefähigen Analyten sowie Reagenz zur Durchführung dieses Verfahrens.**

(30) Priorität: **30.10.90 DE 4034509**

(43) Veröffentlichungstag der Anmeldung:
**06.05.92 Patentblatt 92/19**

(45) Bekanntmachung des Hinweises auf die Patenterteilung:
**22.11.95 Patentblatt 95/47**

(84) Benannte Vertragsstaaten:
**AT BE CH DE DK ES FR GB GR IT LI LU NL SE**

(56) Entgegenhaltungen:
**EP-A- 0 148 463**
**WO-A-85/02258**
**WO-A-89/00694**

**PATENT ABSTRACTS OF JAPAN vol. 7, no. 73 (P-186)(1218)**

**25. März 1983 & JP- A-58 002 660 ( CHUGAI SEIYAKU K.K. ) 8. Januar 1983**

(73) Patentinhaber: **BOEHRINGER MANNHEIM GMBH**

**D-68298 Mannheim (DE)**

(72) Erfinder: **Karl, Johann, Dr.**
**Bert-Schratzlseer-Strasse 7**
**W-8123 Peissenberg (DE)**
Erfinder: **Lang, Fridl, Dr.**
**Herzogstandstrasse 2**
**W-8132 Tutzing (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein immunologisches Präzipitationsverfahren zur Bestimmung eines bindefähigen Analyten durch Inkubation einer Probelösung, die den Analyten enthält, mit einem mit dem Analyten bindefähigen, spezifischen Rezeptor, sowie ein hierfür geeignetes Reagenz.

Immunologische Präzipitationsverfahren werden seit langem zur Bestimmung von bindefähigen Analyten genutzt. Dabei macht man sich die Tatsache zunutze, daß viele immunologische Reaktionen zu Molekülaggregaten oder - im Falle einer Agglutinationsreaktion - zu Partikelaggregaten führen, die sich im Streulichtverhalten stark von den Ausgangsstoffen unterscheiden und deren Konzentration über diese Eigenschaft bestimmt werden kann. Zur quantitativen Bestimmung von Analyten wird die Lichtstreuung an Teilchen, die sich in einem homogenen Medium befinden, sowohl über die Messung der Streulichtintensität (Nephelometrie) als auch über die Messung des Intensitätsverlustes des durch das Medium tretenden Lichtstrahls (Turbidimetrie) genutzt.

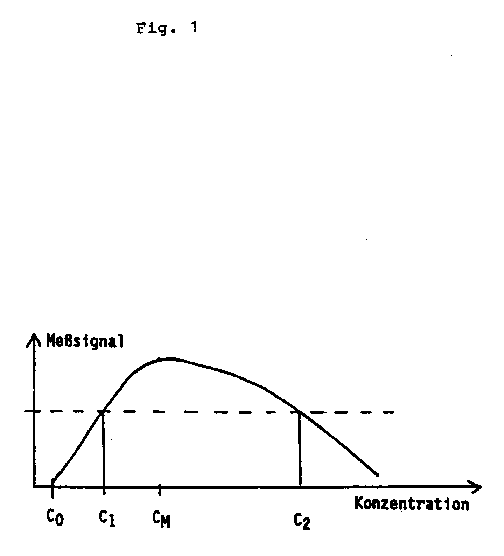

Ein grundsätzliches Problem aller quantitativer immunologischer Präzipitationsverfahren ergibt sich aus der Form der Reaktionskurve. Werden bei konstanter Antikörperkonzentration steigende Antigenmengen zugegeben, so ergibt sich für die Präzipitation ein typischer Kurvenverlauf (Heidelberger Kurve) (Fig. 1). In der Zone des Antikörperüberschusses nimmt die Konzentration des Präzipitats und damit das Meßsignal zu. Bei weiterer Antigenzugabe durchläuft die Kurve ein Maximum und nimmt in der Zone des Antigenüberschusses wieder ab. Aufgrund dieses Effektes können einem Meßsignal zwei Antigenkonzentrationswerte zugeordnet werden. Liegt das Meßsignal im Falle des Antigenüberschusses ($C_2$) innerhalb des Meßbereiches ($C_0$ - $C_M$), für den der aufsteigende Ast der Heidelberger Kurve geeignet ist, so wird die falsche Antigenkonzentration $C_1$, abgelesen. Dieser Effekt wird bei immunologischen Bestimmungsmethoden auch als "High-Dose-Hook-Effekt", im folgenden kurz "Hook-Effekt" genannt, bezeichnet. Bei vielen Analyten, vor allem bei Proteinen, liegt die vorkommende höchstmögliche physiologische Konzentration weit jenseits des Maximums der Heidelberger Kurve, so daß diese Fehlermöglichkeiten sehr häufig auftreten. Um diese Fehler zu vermeiden, muß bei der Bestimmung festgestellt werden, ob das Meßsignal sich im aufsteigenden oder absteigenden Ast der Heidelberger Kurve befindet.

Die älteste und sicherste Methode wird von Schulze, H. E. und G. Schwick, Prot. Biol. Fluids 5, 15 - 25 (1958) beschrieben, die eine Doppelbestimmung mit zwei verschiedenen Probenverdünnungen vorsieht. Im Falle des Antigenüberschusses wird bei der stärker verdünnten Probe ein größeres Meßsignal als bei der konzentrierteren Probe gemessen.

Eine verbesserte Ausführungsform dieses Verfahrens sieht den weiteren Zusatz von Antikörpern vor. Bei Vorliegen eines Antigenüberschusses tritt eine Signalerhöhung ein (T. O. Tiffany et al., Clin. Chem. 20, 1055 - 1061 (1974)). Durch zusätzliche Pipettierung von Antigenmaterial bekannter Konzentration läßt sich ein Antigenüberschuß ebenfalls erkennen (J. C. Sternberg, Clin. Chem. 23, 1456 - 1464 (1977)).

Weiterhin sind mehrere Verfahren beschrieben, die durch aufwendige rechnergesteuerte Auswertungen den Hook-Effekt zu erkennen suchen. Durch Bestimmung der Reaktionsdauer bis zum Auftreten der maximalen Reaktionsgeschwindigkeit bei nephelometrischen Messungen kann zwischen Antigen- und Antikörperüberschuß diskriminiert werden (DE-A-27 24 722; EP-B-0 148 463).

Diese verfahren besitzen alle den Nachteil, daß entweder ein zusätzlicher Pipettierschritt oder eine rechnergesteuerte Auswertung notwendig ist und führen bei der Automatisierung zwangsläufig zu einer Erhöhung des Gerätepreises. Eine bessere Möglichkeit, Fehlinterpretationen bei immunologischen Präzipitationsverfahren zu vermeiden, wäre die Gestalt der Heidelberger Kurve so zu verändern, daß nach dem Maximum ein Plateau erreicht wird und das Auftreten des Hook-Effektes ganz vermieden oder in einem Antigenkonzentrationsbereich verschoben wird, der unter physiologischen Bedingungen nicht mehr auftritt.

In US 4,595,661 werden Sandwich- und nephelometrische Immunoassays beschrieben, die zur Reduzierung des Hook-Effektes neben den normalerweise in einem Immunoassay vorhandenen hochspezifischen Antikörpern und Reagenzien zusätzlich mindestens einen weiteren niedrigaffinen Antikörper gegen den Analyten enthalten. Der Nachteil dieses Verfahrens liegt auf der Hand, da für jeden nachzuweisenden Analyten gleich zwei spezifische Antikörper mit unterschiedlichen Affinitäten zum Analyten hergestellt werden müssen.

WO 89/00694 beschreibt ein Präzipitationsverfahren, wobei Polyethylenglykol mit einem Molekulargewicht von 4.000 bis 20.000 als Reaktionsbeschieuniger eingesetzt wird. In Patent Abstract of Japan. Vol.7, No. 73 (p. 186) (1218) vom 25.03.1983 und JP-A-58 002 660 wird ein Präzipitationsverfahren beschrieben, wobei ein Saccharose-Polymer, u.a. Dextran, mit einem Molekulargewicht von 10.000 bis 500.000 eingesetzt wird. Die Verwendung dieser Polymere zur Vermeidung des Hook-Effektes wird nicht offenbart.

Die Aufgabe der Erfindung war es deshalb, ein einfaches immunologisches Präzipitationsverfahren zur Bestimmung eines bindefähigen Analyten bereitzustellen, das den Hook-Effekt als Störung vermeidet. Die Aufgabe wird durch die in den Ansprüchen näher charakterisierte Erfindung gelöst. Im wesentlichen wird die Aufgabe dadurch gelöst, daß der Testlösung ein nichtionisches Polymer in einer Menge zugesetzt wird, die ausreicht, den Hook-Effekt als Störung zu vermeiden.

Gegenstand der Erfindung ist somit ein immunologisches Präzipitationsverfahren zur Bestimmung eines bindefähigen Analyten durch Inkubation einer Probelösung, die den Analyten enthält mit einem mit dem Analyten bindefähigen, spezifischen Rezeptor, dadurch gekennzeichnet, daß der Testlösung ein nichtionisches Polymer in einer Menge zugesetzt wird, die ausreicht, den Hook-Effekt als Störung zu vermeiden.

Ein weiterer Gegenstand der Erfindung ist ein Reagenz zur Bestimmung eines bindefähigen Analyten in einem immunologischen Präzipitationsverfahren, das zusätzlich zu den für die Immunreaktion üblicherweise erforderlichen Bestandteile ein nichtionisches Polymer in einer Konzentration enthält, die ausreicht, den Hook-Effekt im Testansatz als Störung zu vermeiden. Die nichtionische Polymerkonzentration beträgt mindestens 1 Gew.-% im Testansatz. Unterhalb dieser Konzentration wird die erfindungsgemäße Wirkung nicht mehr erreicht. Die obere Menge ist durch das Auftreten unspezifischer Trübungen bei hohen Polymerkonzentrationen gegeben. Vorzugsweise hat sich eine nichtionische Polymerkonzentration von 2 bis 6, besonders bevorzugt 3 bis 4 Gew.-% im Testansatz erwiesen. Das erfindungsgemäße Reagenz kann als Pulver, Lyophilisat oder als Lösung vorliegen.

Als nichtionische Polymere können beispielsweise Polyethylenglykol, Polyvinylpyrrolidon oder Dextran eingesetzt werden. Nichtionische Polymere sind in unterschiedlichen Polymerisationsgraden, d. h. mit verschiedenen Molekulargewichten, erhältlich. Für die erfindungsgemäße Lösung sind bevorzugt hochmolekulare nichtionische Polymere geeignet, wobei die obere Grenze des Molekulargewichts davon abhängt, ab welchem Molekulargewicht das Polymer im Testansatz nicht mehr ausreichend löslich ist, um erfindungsgemäß wirksam zu sein. Zum Einsatz beim erfindungsgemäßen Verfahren besitzt Polyethylenglykol vorzugsweise ein Molekulargewicht von mehr als 10000, besonders bevorzugt ein Molekulargewicht zwischen 10000 und 300000 und ganz besonders bevorzugt ein Molekulargewicht von 40000. Polyvinylpyrrolidon besitzt vorzugsweise ein Molekulargewicht von mehr als 100000, besonders bevorzugt 360000 bis 750000. Dextran wird vorzugsweise mit einem Molekulargewicht von mehr als 200000, besonders bevorzugt mit einem Molekulargewicht von 500000 bis 1000000 eingesetzt.

Es war bekannt, durch Zugabe von Polymeren, wie Polyethylenglykol, Dextran oder Hyaluronsäure bei immunologischen Präzipitationsverfahren zur Steigerung der Sensitivität und als Reaktionsbeschleuniger einzusetzen. Polyethylenglykol mit einem Molekulargewicht von 6000 wird üblicherweise in einer Konzentration von ungefähr 4 Gew.-% bei der nephelometrischen oder turbidimetrischen Bestimmung einer immunologischen Präzipitationsreaktion eingesetzt (EP-B-0 148 463).

Überraschend war, daß der erfindungsgemäße Zusatz von nichtionischen Polymeren mit den genannten Molekulargewichten und Konzentrationen bei immunologischen Präzipitationsverfahren den Hook-Effekt, der bei hohen Antigenkonzentrationen auftritt, als Störung vermeiden kann, d. h. den Hook-Effekt vermeiden oder in einen Antigenkonzentrationsbereich, der unter physiologischen Bedingungen nicht mehr vorkommt, verschieben kann. Gleichzeitig wird durch diesen Zusatz die Sensitivität und Geschwindigkeit der immunologischen Präzipitationsreaktion gesteigert, oft sogar über das Maß hinaus, das durch den sonst üblichen Zusatz von Polyethylenglykol 6000 erreicht wird. Der Zusatz von Polyethylenglykol 6000 ist somit im erfindungsgemäßen Verfahren nicht mehr notwendig. Eine falsche Antigenkonzentration kann beim erfindungsgemäßen Verfahren nicht mehr gemessen werden, da bei steigenden Antigenkonzentrationen das Meßsignal nach Überschreiten des Maximums der Präzipitationskurve nicht mehr oder erst bei unphysiologisch hohen Antigenkonzentrationen so stark abfällt, daß es wieder im Meßbereich zu liegen kommt. Eine Diskriminierung zwischen dem aufsteigenden und absteigenden Ast der Heidelberger Kurve, wie sie bisher durch einen zusätzlichen Pipettierschritt oder eine rechnergesteuerte Auswertung bei den Verfahren des Standes der Technik notwendig war, entfällt. Das erfindungsgemäße Verfahren kann ohne Mehrkosten im Vergleich zu den bisher üblichen Verfahren durchgeführt werden, da die Verwendung der erfindungsgemäßen nichtionischen Polymere das bisher üblicherweise zugesetzte Polyethylenglykol 6000 ersetzen kann. Zudem können beim automatisierten Einsatz die Gerätekosten gesenkt werden, da zusätzliche Pipettierschritte oder eine aufwendige Auswertung entfallen können.

Unter einem immunologischen Präzipitationsverfahren im Sinne der Erfindung sind alle Reaktionen zwischen immunologischen Rezeptoren und Analyten zu verstehen, die zu einer Trübung der Testlösung infolge der Ausbildung der Analyt-Rezeptor-Komplexe führen. Die Trübung kann dabei durch lichtstreuende Partikel, an die ein Partner der immunologischen Reaktion, d. h. Rezeptor oder Analyt oder Analytanalogon, gebunden ist oder gebunden werden kann, verstärkt werden. In diesem Fall handelt es sich um einen Agglutinationstest. Besonders bevorzugt wird das erfindungsgemäße nichtionische Polymer in immunologischen Präzipitationsverfahren eingesetzt, in denen die Trübung nur durch die Analyt-Rezeptor-Komplexe verursacht werden, die also keine lichtstreuende Partikel enthalten. Weiterhin sind unter immunologischen Präzipitationsverfahren auch Immundiffusionsverfahren, bevorzugt die radiale Immundiffusion, zu verstehen.

Analyt und Rezeptor können im Rahmen des erfindungsgemäßen Verfahrens prinzipiell alle Substanzpaarungen sein, die miteinander bindefähig sind. In dieser Definition sind im Rahmen der Erfindung außer immunologisch bindefähigen Substanzpaaren auch solche Substanzpaarungen eingeschlossen, die sich analog verhalten.

Als bindefähige, spezifische Rezeptoren werden im Sinne der Erfindung Bindepartner des zu analysierenden Analyten verstanden. Bevorzugt werden als Rezeptoren Antikörper oder Antikörperfragmente eingesetzt. Bei den Antikörpern bzw. Antikörperfragmenten kann es sich sowohl um polyklonale als auch monoklonale Antikörper handeln.

Unter einem Analyten werden Substanzen verstanden, die mindestens zwei Epitope, d. h. Bindestellen für den spezifischen Rezeptor, besitzen. Besonders bevorzugt ist das erfindungsgemäße Verfahren geeignet, wenn es sich bei dem Analyt um ein Protein handelt. Der Analyt kann in einer Körperflüssigkeit, wie Plasma, Serum, Urin, Speichel o. ä., oder in einer geeigneten Pufferlösung enthalten sein. Das erfindungsgemäße Verfahren ist beispielsweise besonders zur Bestimmung von Albumin im Urin, zur Bestimmung von Apolipoprotein AI und B im Serum oder Plasma oder zur Bestimmung von Immunglobulinen, Ferritin, Lp(a) oder $\alpha$-1-Mikroglobulin geeignet. In dem Fall, daß es sich bei den Analyten selbst um einen Antikörper handelt, kann als spezifischer Rezeptor das mit diesem Antikörper reagierende Antigen oder aber ein gegen diesen Antikörper gerichteter Anti-Antikörper eingesetzt werden.

Die Messung der Trübung beim erfindungsgemäßen immunologischen Präzipitationsverfahren kann mit geeigneten Geräten sowohl nephelometrisch als auch turbidimetrisch erfolgen. Die Bestimmung der Konzentration des Analyten der Probe erfolgt durch Vergleich mit einem Standard bekannter Analytkonzentrationen.

Unter einem Reagenz zur Durchführung des immunologischen Präzipitationsverfahrens zur Bestimmung eines bindefähigen Analyten in einer Probelösung ist eine Zusammensetzung zu verstehen, die zusätzlich zu den für das immunologische Präzipitationsverfahren erforderlichen Stoffen, wie beispielsweise Hilfsstoffe, Puffersubstanzen oder - im Falle eines Agglutinationstests - mit einem Bindepartner beschichtete Partikel, vornehmlich Latexpartikel, ein nichtionisches Polymer, das erfindungsgemäß wirksam ist, enthält. Die Konzentration dieses nichtionischen Polymers im Reagenz wird dabei so gewählt, daß im Testansatz, d. h. nach Zugabe der Probe zum Reagenz, die Endkonzentration des Polymers 2 bis 6 Gew.-% beträgt.

Die Erfindung wird durch folgende Beispiele näher erläutert:

1. Bestimmung von humanem Albumin im Urin
Bei der Urindiagnostik ist die Bestimmung von Albumin ein wesentliches Kriterium zur Beurteilung von Nierenschädigungen. Die Normalwerte von Albumin im Urin betragen 10 bis 20 mg/l, physiologische Konzentrationen bis zu 20000 mg/l sind möglich. Zur Vermeidung von Fehlinterpretationen sollte der Hook-Effekt bei der Albuminbestimmung ganz vermieden bzw. in den Konzentrationsbereich oberhalb 20000 mg/l verschoben werden. Der normale Meßbereich bei turbidimetrischen Bestimmungen reicht in der Regel nur bis zu einer Albuminkonzentration von 300 mg/l.

Die Versuchsdurchführung sowie die verwendeten Reagenzien sind in den folgenden Beispielen gleich, mit Ausnahme des Zusatzes des nichtionischen Polymers zur Vermeidung des Hook-Effektes als Störung. Folgende Reagenzlösungen werden verwendet:

Lösung 1 (Reaktionspuffer):
50 mmol/l Tris, pH 8,0
1 Gew.-% nichtionisches Detergenz
0,1 Gew.-% Natriumazid

Der Lösung 1 wurden die nichtionischen Polymere bzw. als Vergleich mit dem Verfahren des Standes der Technik PEG 6000 in solchen Konzentrationen zugegeben, daß im Testansatz die jeweils angegebene Endkonzentration erreicht wird.

Lösung 2 (Antiserum):
100 mmol/l Tris, pH 7,2
100 mmol/l NaCl
0,1 Gew.-% Natriumazid
15 mg/ml polykonale anti-humanes Serumalbumin Schafantikörper (PAK<HSA>S-IgG)

Lösung 3 (Kalibratoren):
50 mmol/l Phosphatpuffer, pH 8,0
100 mmol/l NaCl
0,1 Gew.-% Natriumazid
0 bis 20000 mg/l humanes Albumin (HSA)

Die Messungen wurden bichromatisch bei einer Wellenlänge von 340 nm (Korrekturwellenlänge 700 nm) an einem Hitachi 704 der Firma Boehringer Mannheim (Mannheim, Deutschland) bei einer Temperatur von 37° C durchgeführt. 20 $\mu$l Lösung 3 werden mit 350 $\mu$l Lösung 1 vermischt und 5 Minuten inkubiert. Danach erfolgte die erste Messung der Extinktion (E1). 70 $\mu$l

Lösung 2 wurden zupipettiert und der Testansatz weiterhin 5 Minuten inkubiert. Danach erfolgte eine weitere Extinktionsmessung (E2). Zur Auswertung der Ergebnisse wurde die Extinktionsdifferenz ΔE = E2-E1 gegen die Albuminkonzentration graphisch aufgetragen.

1. 1 Vermeidung des Hook-Effektes durch Zugabe von Polyethylenglykol

1.1.1 Vergleich zwischen dem Verfahren des Standes der Technik (Einsatz von PEG 6000) und dem erfindungsgemäßen Verfahren (Einsatz von PEG 40000)

Der Lösung 1 wurde PEG 6000 bzw. PEG 40000 in solchen Konzentrationen zugesetzt, daß im Testansatz eine Endkonzentration von jeweils 4 Gew.-% erreicht wird. Die Testdurchführung erfolgte wie unter Punkt 1 beschrieben.

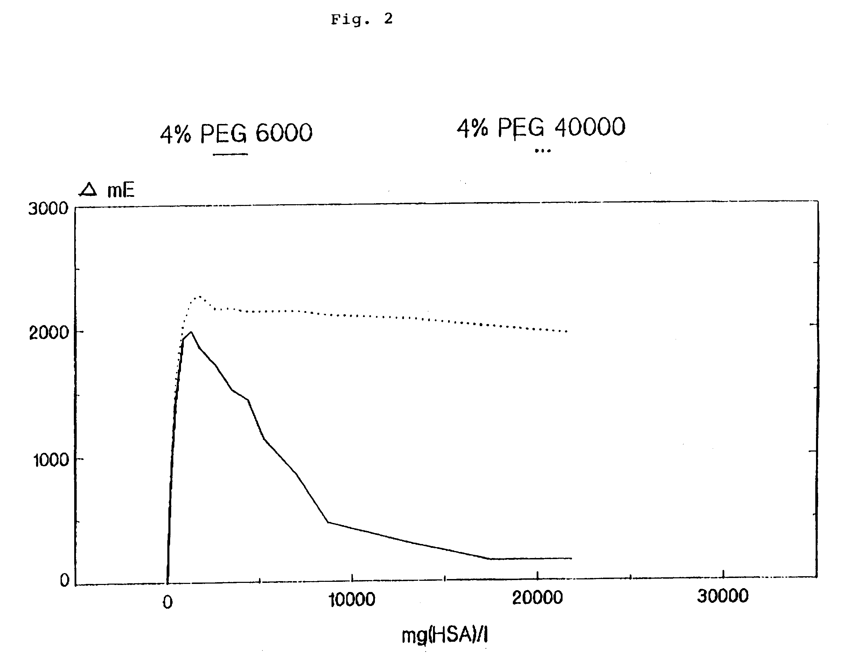

Bei dem Verfahren des Standes der Technik, dem Einsatz von PEG 6000, tritt der Hook-Effekt bei Albuminkonzentrationen oberhalb des Meßbereiches, der sich bis ungefähr 400 mg/l HSA erstreckt, auf. Bei dem erfindungsgemäßen Einsatz von PEG 40000 kommt es bei HSA-Konzentrationen oberhalb des Meßbereiches zu keiner signifikanten Abnahme der Extinktion. Bis zu der unter physiologischen Bedingungen möglichen HSA-Konzentration von ungefähr 20000 mg/l wird der Hook-Effekt als Störung vermieden (Fig. 2). Weiterhin zeigte sich, daß das erfindungsgemäße Verfahren zu einer Reaktionsbeschleunigung und zu einer Steigerung der Sensitivität des immunologischen Präzipitationsverfahrens führte, die über das Maß hinausging, daß durch das Verfahren des Standes der Technik erreicht wird.

1.1.2 Einfluß der Konzentration von PEG 40000

Die Lösung 1 wurde PEG 40000 in unterschiedlichen Konzentrationen zugesetzt. Im Testansatz betrugen die Endkonzentrationen zwischen 0 und 6 Gew.-% . Der Einsatz noch höherer Konzentrationen ist nicht mehr sinnvoll, da oberhalb von 6 Gew.-% PEG 40000 unspezifische Trübungen auftreten, die eine exakte Messung verhindern.

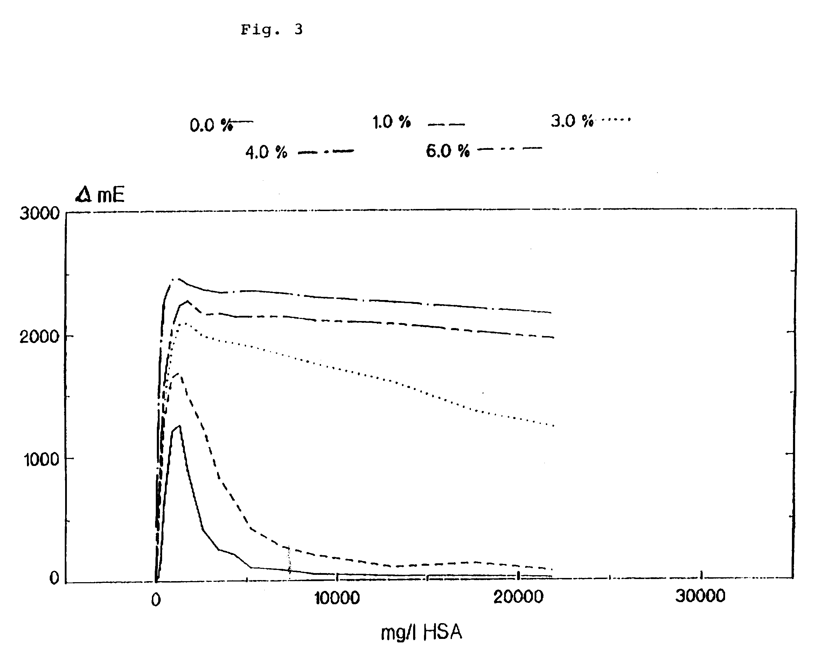

Bei PEG 40000-Konzentrationen bis 1 % tritt der Hook-Effekt oberhalb von HSA-Konzentrationen von 400 mg/l auf (Fig. 3). Oberhalb 1 Gew.-% PEG 40000 ergibt sich eine deutliche Abnahme des Hook-Effektes im Vergleich zur Methode des Standes der Technik (4 Gew.-% PEG 6000 die Werte wurden in Fig. 3 nicht graphisch dargestellt). Aus diesen Ergebnissen ist ersichtlich, daß das nichtionische Polymer erfindungsgemäß in Konzentrationen von mindestens 1, bevorzugt 2 bis 6 Gew.-% und besonders bevorzugt in Konzentrationen von 3 bis 4 Gew.-% eingesetzt werden kann.

1.1.3 Einfluß des Molekulargewichtes von Polyethylenglykol

Um den Einfluß des Molekulargewichtes von PEG auf die immunologische Präzipitation zu ermitteln wurde das Polymer in verschiedenen Polymerisationsgraden eingesetzt. PEG mit einem Molekulargewicht von 2000, 6000, 10000, 40000 und 300000 wurden getestet. Die jeweilige Konzentration im Testansatz betrug einheitlich 4 Gew.-%.

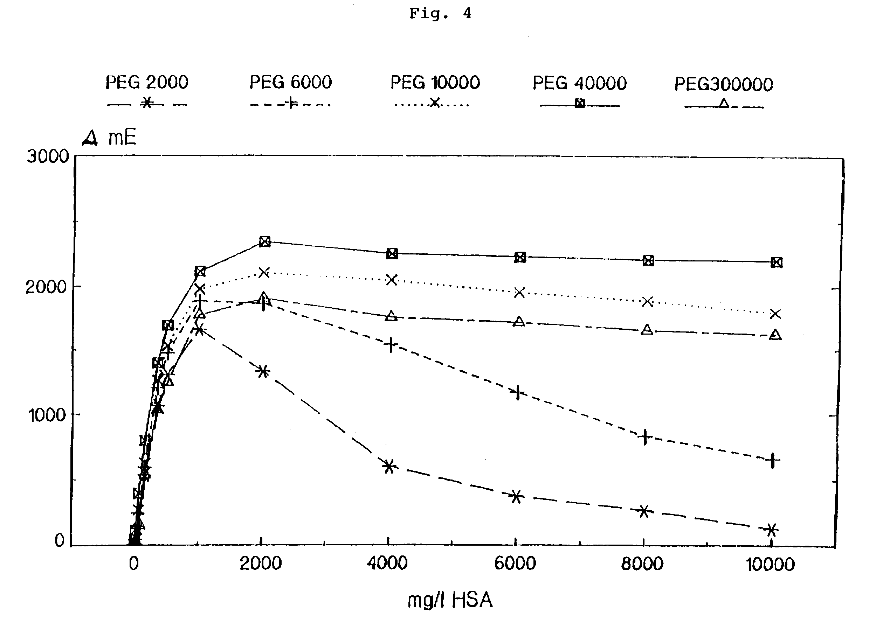

Oberhalb des Molekulargewichts von 10000 wird der Hook-Effekt als Störung vermieden. Besonders bevorzugt wird PEG mit einem Molekulargewicht von 40000 eingesetzt (Fig. 4).

1.2 Bestimmung von Albumin im Urin mit Dextranzusatz

Wie in dem Beispiel 1.1.3 beschrieben, wird der Einfluß des Molelulargewichtes eines weiteren nichtionischen Polymers, in diesem Fall Dextran im erfindungsgemäßen Verfahren am Beispiel der Albuminbestimmung im Urin ermittelt. Die Konzentration der Dextrane im Testansatz beträgt jeweils 4 Gew.-%.

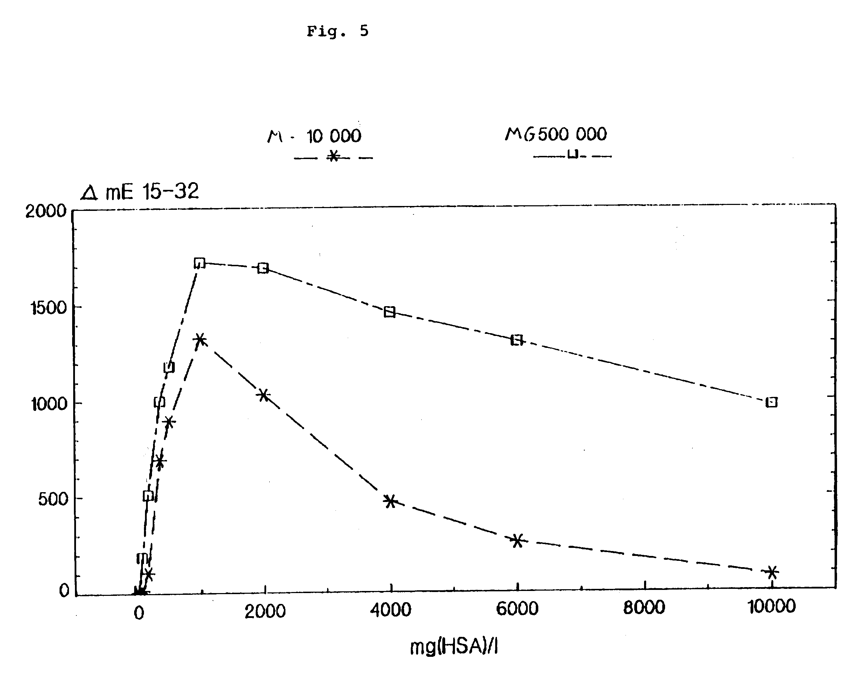

Oberhalb eines Molekulargewichtes von 500000 kann Dextran als Zusatz zur Vermeidung des Hook-Effektes als Störung bei immunologischen Präzipitationsverfahren eingesetzt werden. Dextrane mit einem niedrigen Molekulargewicht erzielten nicht die erfindungsgemäße Wirkung (Fig. 5).

1.3 Bestimmung von Albumin im Urin mit PVP-Zusatz

Am Beispiel der Albuminbestimmung wird der Einfluß des Molekulargewichtes von Polyvinylpyrrolidon (PVP) im erfindungsgemäßen Verfahren bestimmt. PVP wurde jeweils in einer Konzentration von 4 Gew.-% bezogen auf den Testansatz eingesetzt.

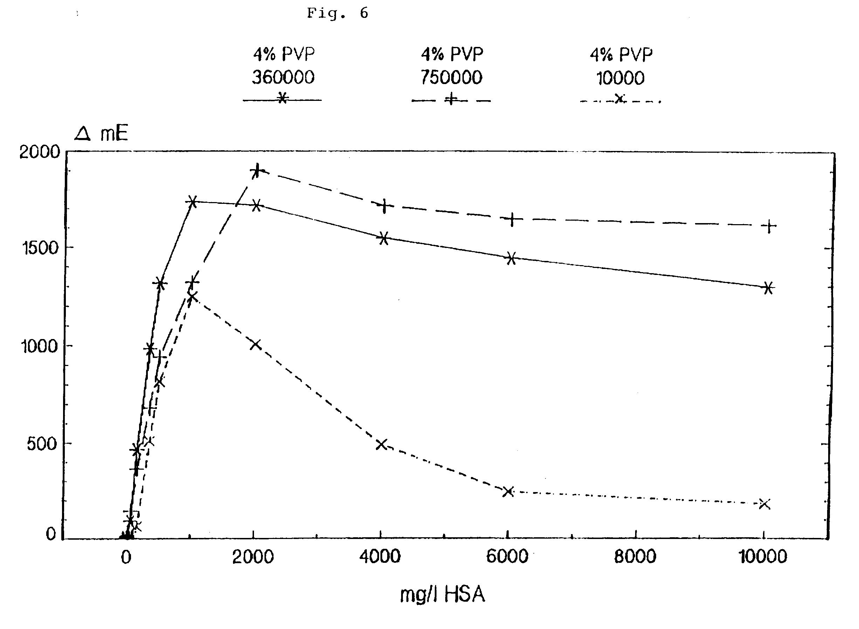

Oberhalb eines Molekulargewichtes von mehr als 100000 kann PVP zur Vermeidung des Hook-Effektes als Störung erfindungsgemäß eingesetzt werden. Wie aus Fig. 6 ersichtlich, tritt bei PVP mit einem Molekulargewicht von 360000 und 750000 keine signifikante Erniedrigung der Extinktion oberhalb des Meßbereiches auf, während PVP mit einem Molekulargewicht von 10000 diese Wirkung nicht erzielt.

2. Bestimmung von Apolipoprotein A-I (Apo A-I)

Bei der Bestimmung der Konzentration von Apolipoprotein A-I wurden die Versuche wie im Beispiel 1 beschrieben durchgeführt. Die Messungen wurden bichromatisch bei einer Wellenlänge von 376 nm (Korrekturwellenlänge 700 nm) an einem Hitachi 704 der Firma Boehringer Mannheim GmbH (Mannheim, Bundesrepublik Deutschland) bei einer Temperatur von 30 °C vorgenommen.

Folgende Reagenzlösungen werden verwendet:

Lösung 1 (Reaktionspuffer):

50 mmol/l Tris, pH 8,0

1 Gew.-% nichtionisches Detergenz

Der Lösung wurde PEG 40000 bzw. als Vergleich mit dem Verfahren des Standes der Technik PEG 6000 in solchen Konzentrationen zugegeben, daß im Testansatz eine Endkonzentration von 4 % erreicht wird.

Lösung 2 (Antiserum):

Es wurde das von der Firma Boehringer Mannheim GmbH unter der Id.Nr. 1381130 erhältliche Antiserum, das polyklonale anti-Apolipoprotein A1-Schafantikörper (PAK<Apo A1>S-IgG) in 100 mmol Tris, pH 7,2 enthält, verwendet.

Lösung 3 (Kalibrator):

Als Kalibrator diente lyophilisiertes Humanserum, das unter der Id.Nr. 1381156 der Firma Boehringer Mannheim GmbH erhältlich ist.

2 $\mu$l Lösung 3 wurden mit 350 $\mu$l Lösung 1 vermischt und fünf Minuten inkubiert. Danach erfolgte die erste Extinktionsmessung (E1). 140 $\mu$l Lösung 2 wurden zupipettiert, der Testansatz weitere fünf Minuten inkubiert und eine zweite Extinktionsmessung (E2) vorgenommen. Zur Auswertung wurde die Extinktionsdifferenz E = E2 - E1 gegen die Apolipoprotein A-I Konzentration grafisch aufgetragen.

Die Eichkurve wurde analog der Packungsbeilage (Id.Nr. 1378686) des Tina-quant® Apolipoprotein A-I Testkits der Firma Boehringer Mannheim GmbH vermessen. Dabei wurde der Kalibrator, der 211 mg Apolipoprotein A-I/dl enthielt, in unterschiedlichen Verdünnungsschritten mit einer 0,9 Gew.-% NaCl-Lösung verdünnt. Die hohen Apolipoprotein A-I-Konzentrationen wurden durch Pipettierung des unverdünnten Kalibrators mit höheren Volumina (2 $\mu$l, 4 $\mu$l etc.) erreicht.

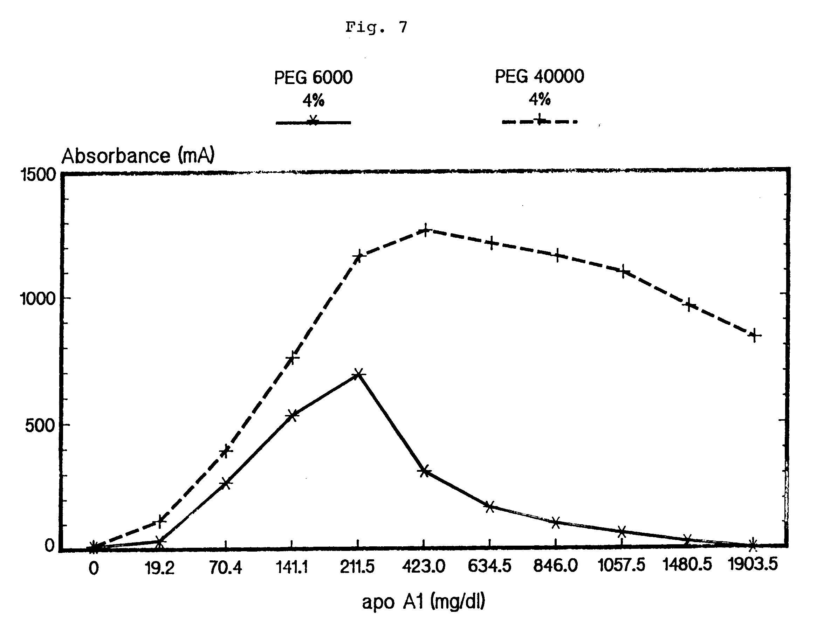

Aus Fig. 7 ist zu entnehmen, daß bei den Verfahren nach dem Stand der Technik, also beim Einsatz von PEG 6000, ein Hook-Effekt oberhalb einer Apo-A-I-Konzentration von ca. 210 mg auftritt. Durch den erfindungsgemäßen Einsatz von PEG 40000 wird der Hook-Effekt in einen weit höheren Konzentrationsbereich verschoben.

3. Bestimmung von $\alpha$-1-Mikroglobulin ($\alpha$-1-M)

Die Messungen wurden wie in den vorangegangenen Beispielen an einem Hitachi 704 durchgeführt. Die Meßtemperatur beträgt 37 °C. Gemessen wurde bei einer Wellenlänge von 340 nm mit einer Korrekturwellenlänge von 700 nm.

Folgende Reagenzlösungen wurden verwendet:

Lösung 1 (Reaktionspuffer): 50 mmol/l Tris, pH 8,0

150 mmol/l NaCl

1 Gew.-% nichtionisches Detergenz PEG 6000 bzw. 40000 in solchen Konzentrationen, daß im Testansatz eine Endkonzentration von 4 % erreicht wird.

Lösung 2 (Antiserum):

20 mg/l polyklonale anti-$\alpha$-1-Mikroglobulin-Schaf-IgG-Antikörper (PAK<$\alpha$-1-M>S-IgG) in 50 mmol/l Tris, pH 8,0

Lösung 3 (Kalibrator):

Als Kalibrator diente ein Urinkonzentrat mit einem $\alpha$-1-Mikroglobulingehalt von 2700 mg/l.

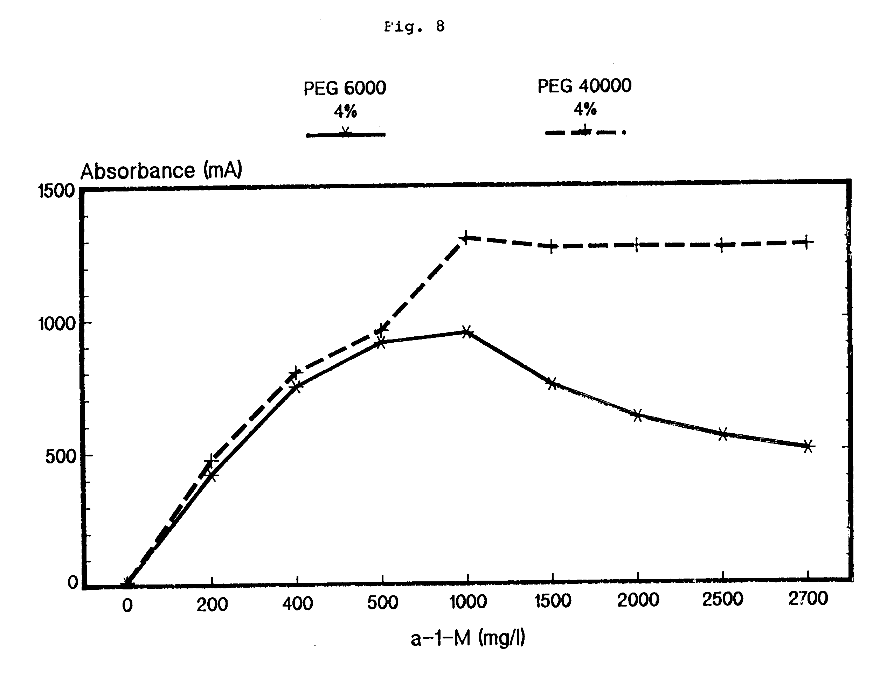

20 $\mu$l Lösung 3 wurden mit 350 $\mu$l Lösung 1 vermischt und nach fünf Minuten die Extinktion (E1) gemessen. Nach Zugabe von 70 $\mu$l Lösung 2 und fünfminütiger weiterer Inkubation wurde die zweite Extinktion (E2) gemessen. Die Extinktionsdifferenz $\Delta$E = E2 - E1 wurde zur Auswertung grafisch aufgetragen (Fig. 8). Zur Erstellung einer Eichgeraden wurde der Kalibrator in 20 mmol/l Hepes verdünnt.

Bei dem Einsatz von PEG 6000 (Verfahren nach dem Stand der Technik) wurde oberhalb einer $\alpha$-1-M-Konzentration von 1000 mg/l ein Hook-Effekt beobachtet. Beim Einsatz von PEG 40000 konnte über den gesamten Meßbereich bis zu einer Konzentration von 2700 mg/l kein Hook-Effekt beobachtet werden.

**Patentansprüche**

1. Immunologisches Präzipitationsverfahren zur Bestimmung eines bindefähigen Analyten durch Inkubation einer Probelösung, die den Analyten enthält, mit einem mit dem Analyten bindefähigen, spezifischen Rezeptor, dadurch gekennzeichnet, daß der Testlösung ein nichtionisches Polymer aus der Gruppe: Polyvinylpyrrolidon, mit einem Molekulargewicht von mehr als 100.000 und Polyethylenglycol, mit einem Molekulargewicht von 40.000 bis 300.000, zugesetzt wird.

2. Verfahren nach Anspruch 1, dadurch gekennzeichnet, daß das nichtionische Polymer in einer Konzentration von mindestens 1 Gew.-% im Testansatz vorliegt.

3. Verfahren nach Anspruch 1, dadurch gekennzeichnet, daß das nichtionische Polymer in einer Konzentration von 2 bis 6 Gew.-% im Testansatz vorliegt.

4. Verfahren nach einem der Ansprüche 1 bis 3, dadurch gekennzeichnet, daß Polyvinylpyrrolidon mit einem Molekulargewicht von 360.000 bis 750.000 verwendet wird.

5. Verfahren nach einem der Ansprüche 1 bis 4, dadurch gekennzeichnet, daß es sich um einen Agglutinationstest handelt.

6. Verfahren nach einem der Ansprüche 1 bis 5, dadurch gekennzeichnet, daß der spezifische Rezeptor ein polyklonaler Antikörper ist.

7. Verfahren nach einem der Ansprüche 1 bis 6, dadurch gekennzeichnet, daß der spezifische Rezeptor ein Gemisch aus mindestens zwei monoklonalen Antikörpern ist.

8. Verfahren nach einem der Ansprüche 1 bis 7, dadurch gekennzeichnet, daß der Analyt ein Protein ist.

9. Verfahren nach einem der Ansprüche 1 bis 8, dadurch gekernzeichnet, daß der Analyt Albumin, Apolipoprotein A I oder $\alpha$-1-Microglobulin ist.

10. Reagenz zur Durchführung eines immunologischen Präzipitationsverfahrens zur Bestimmung eines bindefähigen Analyten in einer Probelösung, dadurch gekennzeichnet, daß es zusätzlich zu den für das immunologische Präzipitationsverfahren erforderlichen Stoffen ein nichtionisches Polymer aus der Gruppe: Polyvinylpyrrolidon, mit einem Molekulargewicht von mehr als 100000 und Polyethylenglycol, mit einem Molekulargewicht von 40.000 bis 300.000 enthält.

11. Reagenz gemäß Anspruch 10, dadurch gekennzeichnet, daß die nichtionische Polymerkonzentration mindestens 1 Gew.-% im Testansatz beträgt.

12. Reagenz gemäß Anspruch 10, dadurch gekennzeichnet, daß die nichtionische Polymerkonzentration 2 bis 6 Gew.-% im Testansatz beträgt.

13. Verwendung eines nichtionischen Polymers aus der Gruppe: Dextran, mit einem Molekuargewicht von mehr als 500.000, Polyvinylpyrrolidon, mit einem Molekuargewicht von mehr als 100.000 und Polyethylenglycol, mit einem Molekuargewicht von mehr als 10.000, zugesetzt wird zur Vermeidung des Hook-Effekts als Störung bei immunologischen Präzipitationsverfahren.

## Claims

1. Immunological precipitation process for the determination of a bindable analyte by incubation of a sample solution which contains the analyte with a specific receptor bindable with the analyte, characterised in that to the test solution is added a non-ionic polymer from the group: polyvinylpyrrolidone with a molecular weight of more than 100,000 and polyethylene glycol with a molecular weight of 40,000 to 300,000.

2. Process according to claim 1, characterised in that the non-ionic polymer is present in the test batch in a concentration of at least 1 wt.%.

3. Process according to claim 1, characterised in that the non-ionic polymer is present in the test batch in a concentration of 2 to 6 wt.%.

4. Process according to one of claims 1 to 3, characterised in that polyvinylpyrrolidone is used with a molecular weight of 360,000 to 750,000.

5. Process according to one of claims 1 to 4, characterised in that it is a question of an agglutination test.

6. Process according to one of claims 1 to 5, characterised in that the specific receptor is a polyclonal antibody.

7. Process according to one of claims 1 to 6, characterised in that the specific receptor is a mixture of at least two monoclonal antibodies.

8. Process according to one of claims 1 to 7, characterised in that the analyte is a protein.

9. Process according to one of claims 1 to 8, characterised in that the analyte is albumin, apolipo-protein A-I or $\alpha$-1-microglobulin.

10. Reagent for the carrying out of an immunological precipitation process for the determination of a bindable analyte in a sample solution, characterised in that, in addition to the material necessary for the immunological precipitation process, it contains a non-ionic polymer from the group: polyvinylpyrrolidone with a molecular weight of mote than 100,000 and polyethylene glycol with a molecular weight of 40,000 to 300,000.

11. Reagent according to claim 10, characterised in that the non-ionic polymer concentration amounts to at least 1 wt.% in the test batch.

12. Reagent according to claim 10, characterised in that the non-ionic polymer concentration amounts to 2 to 6 wt.% in the test batch.

**13.** Use of a non-ionic polymer from the group: dextran with a molecular weight of more than 500,000, polyvinylpyrrolidone with a molecular weight of more than 100,000 and polyethylene glycol with a molecular weight of more than 10,000 is added for the avoidance of the hook effect as disturbance in immunological precipitation processes.

## Revendications

**1.** Procédé de précipitation immunologique pour la détermination d'un analyte capable de liaison, par incubation d'une solution d'échantillon qui contient l'analyte, avec un récepteur spécifique, capable d'entrer en liaison avec l'analyte, caractérisé en ce qu'on ajoute à la solution de test un polymère non ionique du groupe comprenant la polyvinylpyrrolidone, ayant une masse moléculaire supérieure à 100 000 et le polyéthylèneglycol, ayant une masse moléculaire de 40 000 à 300 000.

**2.** Procédé selon la revendication 1, caractérisé en ce que le polymère non ionique est présent dans le mélange à analyser en une concentration d'au moins 1 % en poids.

**3.** Procédé selon la revendication 1, caractérisé en ce que le polymère non ionique est présent dans le mélange à analyser en une concentration de 2 à 6 % en poids.

**4.** Procédé selon l'une quelconque des revendications 1 à 3, caractérisé en ce que l'on utilise une polyvinylpyrrolidone ayant une masse moléculaire de 360 000 à 750 000.

**5.** Procédé selon l'une quelconque des revendications 1 à 4, caractérisé en ce qu'il s'agit d'un test d'agglutination.

**6.** Procédé selon l'une quelconque des revendications 1 à 5, caractérisé en ce que le récepteur spécifique est un anticorps polyclonal.

**7.** Procédé selon l'une quelconque des revendications 1 à 6, caractérisé en ce que le récepteur spécifique est un mélange d'au moins deux anticorps monoclonaux.

**8.** Procédé selon l'une quelconque des revendications 1 à 7, caractérisé en ce que l'analyte est une protéine.

**9.** Procédé selon l'une quelconque des revendications 1 à 8, caractérisé en ce que l'analyte est l'albumine, l'apolipoprotéine A I ou l'$\alpha$-1-microglobuline.

**10.** Réactif pour la mise en oeuvre d'un procédé de précipitation immunologique pour la détermination d'un analyte dans une solution d'échantillon, caractérisé en ce qu'en plus des substances nécessaires pour le procédé de précipitation immunologique, il contient un polymère non ionique du groupe comprenant la polyvinylpyrrolidone, ayant une masse moléculaire supérieure à 100 000 et le polyéthylèneglycol, ayant une masse moléculaire de 40 000 à 300 000.

**11.** Réactif selon la revendication 10, caractérisé en ce que la concentration du polymère non ionique est d'au moins 1 % en poids dans le mélange à analyser.

**12.** Réactif selon la revendication 10, caractérisé en ce que la concentration du polymère non ionique est de 2 à 6 % en poids dans le mélange à analyser.

**13.** Utilisation d'un polymère non ionique du groupe comprenant le dextrane, ayant une masse moléculaire supérieure à 500 000, la polyvinylpyrrolidone, ayant une masse moléculaire supérieure à 100 000 et le polyéthylèneglycol, ayant une masse moléculaire supérieure à 10 000, ajouté pour éviter l'effet de Hook comme perturbation du procédé de précipitation immunologique.

Fig. 1

Fig. 2

Fig. 3

Fig. 4

Fig. 5

Fig. 6

EP 0 483 512 B1

Fig. 7

Fig. 8